# EUROPEAN PATENT SPECIFICATION

(11) **EP 3 441 108 B1**
(45) Date of publication and mention of the grant of the patent: **28.07.2021**
(21) Application number: 18187954.5
(22) Date of filing: 08.08.2018
(51) Int. Cl.: A61N 1/04, A61B 5/00

(54) **APPARATUS FOR TESTING PERCEPTION**
VORRICHTUNG ZUR PRÜFUNG DER WAHRNEHMUNG
APPAREIL DE TEST DE PERCEPTION

(30) Priority: 10.08.2017 JP 2017155398
(43) Date of publication of application: 13.02.2019
(73) Proprietor: Nihon Kohden Corporation, Shinjuku-ku Tokyo (JP)
(72) Inventor: Kaiami, Takashi, Saitama (JP); Yoshida, Ryoko, Saitama (JP); Motogi, Jun, Saitama (JP)
(74) Representative: Hoffmann Eitle

(56) References cited:
- WO-A1-2016/146758
- US-A- 5 806 522
- US-A1- 2015 148 865

## Description

### BACKGROUND

The presently disclosed subject matter relates to an apparatus for testing perception. Conventionally, in a peripheral nerve test, a perception test in which the pain nerve is stimulated by supplying a stimulation signal between two electrodes or a needle electrode and a contact electrode is performed (for example, JP2011-164879A).

In a perception test performed on such pain nerve or like peripheral nerve, however, sensory perception of electrical stimulation pain varies among individuals. Because of the nature of a perception test, the test is performed by an application of very little stimulation. Depending on a subject, even when electrical stimulation is applied for a perception test, therefore, the subject may sometimes feel itchy, not recognize pain, and express no sensation of pain.

As a countermeasure against the behavior that, despite perception of electrical stimulation, a subject does not express a sensation of pain, learning of electrical stimulation is performed on a subject. The learning of electrical stimulation is performed in the following manner. Before a perception test, an inspector such as a medical technologist or a nurse applies electrical stimulation of the same kind as that which is to be applied in the perception test, to the subject, thereby causing the subject to actually feel how the electrical stimulation that is to be recognized as pain acts.

However, it is often that electrical stimulation which is to be applied before a perception test is informed to the subject by ambiguous verbal instructions of "You will feel a prick." When the subject begins to receive electrical stimulation, therefore, it is difficult for the subject to know the timing when electrical stimulation is output, or whether the suffered pain is originated from the electrical stimulation or not. In some cases, consequently, the subject cannot perceive the electrical stimulation, and the electrical stimulation is applied several times to the subject. The subject to whom stimulations are applied an excessive number of times as described above becomes insensitive to pain, and consumes the concentration power in the learning of pain, with the result that, in an actual perception test, the test cannot be performed adequately and efficiently.

US 2015/148865 A1 describes an apparatus for transcutaneous electrical nerve stimulation in humans that delivers electrical currents across the intact skin of a patient via electrodes so as to provide symptomatic relief of chronic pain.

US 5 806 522 A describes a method of quantitatively determining and recording constant alternating current conscious perception threshold or current conscious pain perception threshold.

WO 2016/146758 A1 describes a method for assessing nerve fiber excitability.

The presently disclosed subject matter may provide an apparatus for applying electrical stimulation for learning to a subject.

### SUMMARY

This object is accomplished by the subject-matter of the independent claims.

The dependent claims concern particular embodiments.

### BRIEF DESCRIPTION OF THE DRAWINGS

Fig. 1 is a conceptual block diagram of an apparatus for testing perception according to an embodiment of the presently disclosed subject matter.
Fig. 2 is a flowchart of a stimulation learning mode in the embodiment of the presently disclosed subject matter.

### DETAILED DESCRIPTION OF EXEMPLARY EMBODIMENTS

Hereinafter, an embodiment of the presently disclosed subject matter will be described with reference to the drawings.

With an apparatus for testing perception, in order to allow a subject to adequately learn electrical stimulation which is to be applied to the subject in a perception test, electrical stimulation of the same kind as that of the electrical stimulation that is to be applied in the perception test is applied to the subject in advance of execution of the perception test.

The apparatus for testing perception (hereinafter, often referred to as the perception test apparatus) 1 includes a switch 20 which is operated by the subject who, for example, grasps the switch, and a main unit 30 which is connected to the switch 20. The switch 20 includes a light emitter 21 (an example of a visual notification section) which notifies the subject of an application of electrical stimulation. The perception test apparatus 1 is configured so as to be attachable to and detachable from an electrode 10 (an example of the member which applies stimulation to the subject (the stimulation application member), and alternatively a stimulation probe or the like may be used).

The electrode 10 may have any one of various configurations which apply electrical stimulation to the subject. The same as or similarly with JP2011-164879A, for example, the electrode 10 may be configured by a first electrode which is to be used while the tip end is slightly inserted into the skin of the subject, and a second electrode which is placed in the circumference of the first electrode, and which is to be used while being in contact with the skin of the subject. Alternatively, the electrode 10 may be configured by a pair of electrodes which are disposed on a surface that is one surface of a sheet-like sensor to be used while being bonded to the body surface of the subject, and that is to be in contact with the body surface.

The light emitter 21 is the notification section which notifies the subject of an application of electrical stimulation by means of light. For example, the light emitter 21 is configured so that a light emitting diode (LED) emits light.

The main unit 30 has a controller 40, a displaying section 50, a manual inputting section 60, and a power source section 70. For example, the main unit 30 has a configuration in which the displaying section 50, and the controller 40 that performs a perception test function in the perception test apparatus 1 are integrated with each other.

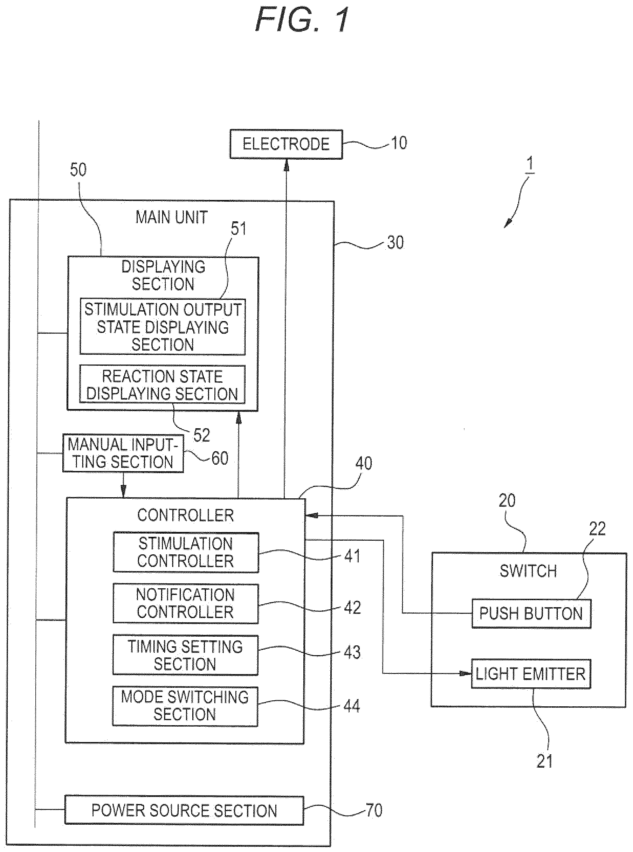

As shown in Fig. 1, the controller 40 has a stimulation controller 41, a notification controller 42, a timing setting section 43, and a mode switching section 44.

The stimulation controller 41 is configured so as to control the output of electrical stimulation from the electrode 10. That is, the stimulation controller 41 is configured so as to set the beginning of the application of electrical stimulation, the ending of the application of electrical stimulation, the output intensity of the electrical stimulation, and the like. The output intensity of the electrical stimulation may be set by using a voltage which is to be applied to the electrode 10, or a current of the electrical stimulation which is output from the electrode 10.

The stimulation controller 41 is further configured so as to change the output intensity of the electrical stimulation. The output intensity may be automatically or manually changed. In the case where the output intensity is automatically changed, a configuration may be employed where a set value relating to a change of the output intensity is previously registered in the stimulation controller 41, and the stimulation controller 41 automatically changes, by using the set value, the set value of the output intensity of the electrical stimulation which is to be next applied. With respect to the set value relating to a change of the output intensity, for example, the increasing/decreasing unit of the current value which is to be increased or decreased can be set to a unit of 0.01 mA.

The notification controller 42 is configured so as to control an output of the notification to the subject which is performed by the notification section (in the embodiment, the light emitter 21). Namely, the notification controller 42 is configured so as to control the light emission by the light emitter 21 in various manners in which the subject can sense the light emission as the notification. The notification controller 42 may control the light emission by the light emitter 21 in a manner that the light emitter is lighted on one time, and immediately thereafter lighted off, that the light emission is continued, or that the light emitter is lighted a plurality of times with respect to one application of electrical stimulation.

The timing setting section 43 is configured so that a timing when the stimulation controller 41 causes electrical stimulation to be output from the electrode 10, and that when the notification controller 42 causes the notification section to output notification can be set. The time interval between the timing when the notification section performs notification, and that when electrical stimulation is applied by the stimulation controller 41 can be changed by the timing setting section 43. A configuration where the time interval between the timing when notification is performed, and that when electrical stimulation is applied is automatically changed may be employed. Alternatively, a configuration where the time interval is manually changed may be employed. In the case where the timing of the output by the electrode 10, and that of the notification by the light emitter 21 are automatically changed, set values relating to random timings such as a random number table may be used. A configuration where the timing setting section 43 previously registers the set values relating to random timings may be employed.

The mode switching section 44 is configured so as to switch a stimulation learning mode and a perception test mode. In the perception test mode, electrical stimulation is applied without performing notification by the notification section, and it is determined whether perception of the electrical stimulation exists or not, thereby testing the peripheral nerve. The stimulation learning mode is a mode in which, when, in advance of a perception test, electrical stimulation of the same kind as that of the electrical stimulation that is to be used in the perception test is to be applied to the subject, the notification section notifies the subject of the application of electrical stimulation.

For example, the mode switching section 44 may have a configuration where, in response to manually input instructions for ending the stimulation learning mode, the stimulation learning mode is ended, and the perception test mode is started.

As shown in Fig. 1, the displaying section 50 has a stimulation output state displaying section 51 and a reaction state displaying section 52. The displaying section 50 may be configured by a touch panel in which an inputting operation can be performed by direct contact of the finger or the like with the panel, or a display which performs only a displaying operation.

The stimulation output state displaying section 51 is configured so as to display various kinds of information relating to electrical stimulation, such as the set value of the current of electrical stimulation which is applied from the electrode 10 to the subject. For example, the stimulation output state displaying section 51 displays characters such as "NOW OUTPUTTING" and "ALREADY OUTPUTTED," a graphic symbol such as a lightning symbol indicating that electrical stimulation is outputting, the output intensity (the current, the applied voltage) of the electrical stimulation, and the like.

The reaction state displaying section 52 is configured so as to display an expression that, in response to an application of electrical stimulation, the subject perceives the electrical stimulation. For example, the reaction state displaying section 52 displays a numerical value indicating the number of seconds of the reaction time extending from the timing when the electrical stimulation is applied, to expression by the subject of perception, graphic symbols such as a face symbol indicating pain, and the like.

The displaying section 50 may have a configuration where various kinds of information necessary in the perception test apparatus 1 are displayed, in addition to the stimulation output state displaying section 51 and the reaction state displaying section 52. For example, the displaying section may be configured so as to display contents which are input through the manual inputting section 60.

The manual inputting section 60 is configured so that various set values relating to electrical stimulation and notification can be manually input. The manual inputting section 60 may be configured by operation buttons (not shown) which are disposed on the main unit 30, or a manual input icon (not shown) which is displayed on the displaying section 50 in the case where the displaying section 50 is configured by a touch panel.

The manual inputting section 60 is further configured so that contents relating to settings in the stimulation controller 41, the notification controller 42, the timing setting section 43, and the mode switching section 44 can be input. For example, a current value relating to a change of the output intensity of electrical stimulation, and the like may be input through the manual inputting section 60. As settings in the notification controller 42, settings of the light emission such as the number, colors, intensities, and blinks of light emissions of the light emitter 21 may be input through the manual inputting section 60. As settings in the timing setting section 43, the timing of the output from the electrode 10, and that of notification from the light emitter 21 may be input through the manual inputting section 60.

The perception test apparatus 1 further includes an operating section which, in response to an application of electrical stimulation, is to be operated by the subject in order to inform the inspector of perception of the electrical stimulation. Fig. 1 shows a push button 22 as an example of the operating section.

The shape and size of the push button 22 may be set to any one of various shapes and sizes which allow the subject to operate the button. For example, the shape and size of the switch 20 may be formed by a spindle-like shape having a size at which the switch 20 is slightly projected from the hand of the subject grasping the switch 20. The projected portion of the switch 20 may be configured as the push button 22 which can be pushed by the subject, and also as the light emitter 21.

The power source section 70 is configured so as to supply electric power required for the operations of the switch 20, the light emitter 21, the push button 22, the main unit 30, the controller 40, the displaying section 50, and the manual inputting section 60.

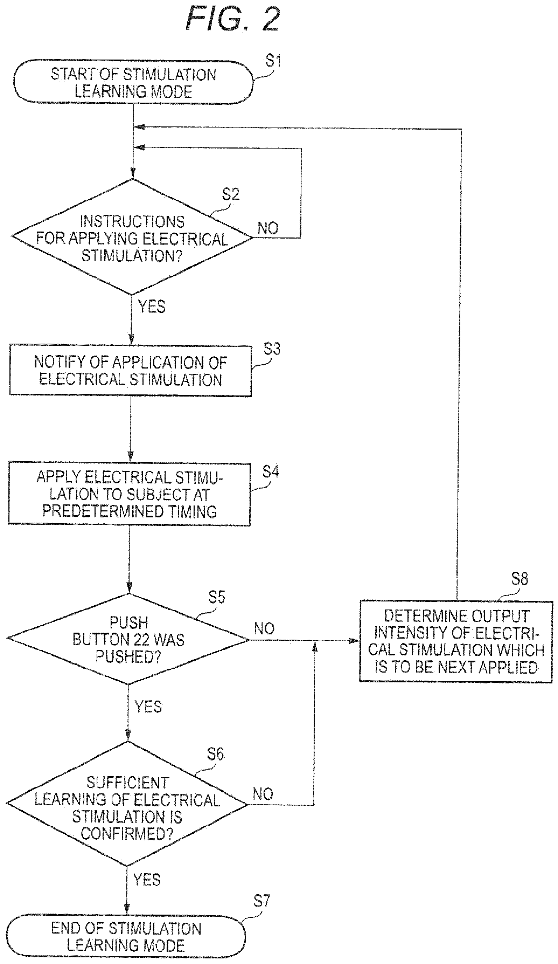

Next, an example of a method for testing perception (hereinafter, often referred to as the perception test method) in which the thus configured perception test apparatus 1 is used will be described with reference to Figs. 1 and 2.

When the inspector performs an input operation for setting the stimulation learning mode, through the manual inputting section 60, the perception test apparatus 1 is set to the stimulation learning mode by the mode switching section 44, and the stimulation learning mode is started (S1).

Then, the inspector operates the manual inputting section 60 to input a setting relating to an application of electrical stimulation, and that relating to notification. In the example, 0.10 mA is input as the output intensity of electrical stimulation through the manual inputting section 60. The settings such as the current value of electrical stimulation which are input through the manual inputting section 60 are set in the stimulation controller 41. In the example, moreover, a setting that the light emitter 21 performs one time the light emission is input through the manual inputting section 60. The color of the light emission of the light emitter 21, and the like which are input through the manual inputting section 60 are set in the notification controller 42.

The inspector inputs a setting of a timing relating to the application of electrical stimulation, and that of a timing relating to notification, through the manual inputting section 60. In the example, the timing setting section 43 performs an input in which a timing of an application of electrical stimulation is set to be 1.0 second after an input of instructions for applying electrical stimulation. The timing setting section 43 further performs an input in which the timing of light emission is set to be minus 0.5 seconds from the timing of an application of electrical stimulation. The timings which are input by using the manual inputting section 60, and which relates to notification and electrical stimulation are set in the timing setting section 43.

After the settings of electrical stimulation and notification are completed, the inspector inputs instructions for applying electrical stimulation, through the manual inputting section 60 (YES in S2). When the instructions for applying electrical stimulation are input, the notification controller 42 outputs, in accordance with the settings in the timing setting section 43, instructions for instructing the light emitter 21 to emit light in a preset notification manner, to cause the light emitter 21 to emit light. In S3, the light emitter 21 emits light one time after 0.5 seconds from the instructions for applying electrical stimulation (S3, an example of the notifying step).

Moreover, the stimulation controller 41 outputs instructions for causing the electrode 10 to output electrical stimulation at the output intensity which is set in the stimulation controller 41, and electrical stimulation is applied from the electrode 10 to the subject at a predetermined timing according to the setting in the timing setting section 43 (at the setting which is set by the timing setting section 43). In S4, after 0.5 seconds from the light emission of the light emitter 21, electrical stimulation at an output intensity of 0.10 mA is applied to the subject by the stimulation controller 41 (S4, an example of the stimulation applying step).

The subject who perceives the application of electrical stimulation pushes the push button 22 to express the perception of electrical stimulation (YES in S5). The expression of perception by the subject is displayed on the reaction state displaying section 52 through the push button 22, and transmitted to and confirmed by the inspector. The inspector confirms that the subject sufficiently learns perception of electrical stimulation (YES in S6), and inputs instructions for ending the stimulation learning mode, through the manual inputting section 60. In response to the instructions for ending the stimulation learning mode, the mode switching section 44 ends the stimulation learning mode (S7), and, upon ending of the stimulation learning mode, switches the perception test apparatus 1 to the perception test mode. When the perception test apparatus 1 is switched to the perception test mode, the inspector continuously performs the perception test on the subject by using the perception test apparatus 1.

Next, the case where the output intensity of electrical stimulation is to be changed will be described with reference to Figs. 1 and 2. A duplicative description of operations which are identical with those that have been described above is omitted.

If, even when electrical stimulation is applied from the electrode 10 in S4 shown in Fig. 2, the subject cannot perceive the application of electrical stimulation, an operation corresponding to the applied electrical stimulation is not performed on the operating section. Namely, the push button 22 is not pushed (NO in S5), and a display indicating that the operating section is operated is not performed on the reaction state displaying section 52. The inspector confirms that further learning of electrical stimulation is necessary for the subject, and causes second and subsequent application of electrical stimulation for learning to be performed.

In second and subsequent application of electrical stimulation for learning, the inspector changes the setting so as to raise the output intensity of electrical stimulation. If an operation corresponding to the electrical stimulation which is first applied is not performed on the operating section (NO in S5), it is considered that the subject cannot recognize the electrical stimulation because the output intensity of the electrical stimulation is lower with respect to the pain sensation of the subject. Therefore, the output intensity of the electrical stimulation which is to be next applied to the subject is raised so that, in the learning of electrical stimulation, the output intensity is at a level at which the subject can recognize the electrical stimulation.

In the learning of second and subsequent application of electrical stimulation, the inspector checks the setting relating to the firstly applied electrical stimulation, through the display on the stimulation output state displaying section 51. The inspector determines the output intensity of electrical stimulation which is to be next applied, based on information of the firstly applied electrical stimulation which is displayed on the stimulation output state displaying section 51 (S8).

The inspector operates the manual inputting section 60 to input the output value of the current which is to be supplied to the electrode 10 that is to be used in the next application. Electrical stimulation which is excessively strong is not good for the subject. When such stimulation is repeatedly applied, the stimulation is hardly perceived because the subject is accustomed to strong stimulation. On the other hand, when the subject undergoes a perception test in a state where the subject cannot perceive applied electrical stimulation, it is impossible to obtain an appropriate result of the perception test. On the basis of these circumstances, the value of an output intensity which is adequate as electrical stimulation for learning is determined. In the example, the inspector confirms that the output intensity of the electrical stimulation which is first applied to the subject is 0.10 mA, through the display on the stimulation output state displaying section 51, and inputs a current value of 0.11 mA as the output intensity of the electrical stimulation which is to be next applied, through the manual inputting section 60.

The set value which is input by the inspector through the manual inputting section 60 is set in the stimulation controller 41 as the set value of the stimulation controller 41 relating to electrical stimulation which is to be next applied. The timing of notification by the notification section, the timing of application of electrical stimulation, and the setting of the notification controller 42 may be identical with those in the first setting, or different setting contents relating them may be newly set.

After the setting of the electrical stimulation which is to be next applied, and that of notification are completed, the inspector inputs instructions for applying the electrical stimulation, through the manual inputting section 60 (YES in S2). When the instructions for applying the electrical stimulation is input, the light emitter 21 emits light in accordance with the setting of the timing setting section 43 (S3), and the electrical stimulation is applied by the electrode 10 (S4).

The subject who perceives the application of electrical stimulation pushes the push button 22 to express the perception of electrical stimulation (YES in S5). The inspector confirms that the subject sufficiently learns perception of electrical stimulation (YES in S6), and causes the stimulation learning mode to be ended, through the manual inputting section 60, and causes the mode to be switched to the perception test mode.

Returning to S5 of Fig. 2, if the application of electrical stimulation is not perceived, and the subject does not operate the operating section (NO in S5), the inspector determines the necessity of continuation of the stimulation learning mode. If the inspector determines that it is necessary to continue the stimulation learning mode, the inspector checks the output intensity of the secondly applied electrical stimulation, and determines the output intensity of the third electrical stimulation which is to be next applied (S8). The operations after S8 are identical with those which have been described above, and therefore their description is omitted.

If the inspector determines that the subject does not sufficiently learn perception of electrical stimulation (NO in S6), the inspector determines the necessity of continuation of the stimulation learning mode. If the inspector determines that it is necessary to continue the stimulation learning mode, electrical stimulation for learning is further applied. The operations which are performed in the further application of electrical stimulation for learning are the same as or similar to those of the second and subsequent electrical stimulations which have been described above, and therefore their description is omitted.

As described above, the perception test apparatus 1 includes: the stimulation controller 41 which controls the electrode 10 that applies electrical stimulation to the subject; and the notification section (in the embodiment, the light emitter 21) which notifies the subject of an application of the electrical stimulation by means of light emission, and therefore can inform the subject of the output of electrical stimulation for learning, regardless of the skill of the inspector. Consequently, an application of electrical stimulation for learning, which in the prior art is informed by ambiguous instructions, can be informed more clearly, and a perception test can be performed more appropriately. The subject to whom electrical stimulation for learning is applied can recognize the kind of pain which is produced by the electrical stimulation that is to be used in a perception test. Therefore, a perception test can be efficiently performed in a reduced number of times of application of electrical stimulation as compared with the case where the learning of electrical stimulation is not performed.

In the case where the subject in whom the final result of the perception test is 0.1 mA does not undergo the learning of electrical stimulation, namely, the current value of electrical stimulation is set to be higher (for example, 1.0 mA) in the perception test mode, and electrical stimulation is applied while gradually lowering the current value, with the result that the number of application of electrical stimulation is increased.

In the case where the learning of electrical stimulation is performed, by contrast, the inspector can surely set the current value at which the subject can perceive electrical stimulation. In the case where the current value at which the subject can perceive electrical stimulation in the stimulation learning mode is 0.3 mA, for example, the current value in the perception test mode can be gradually lowered from a current value (0.3 mA) which is in the vicinity of the minimum current value which can be perceived by the subject. In the case where the learning of electrical stimulation is performed, therefore, it is possible to reduce the number of times of application of electrical stimulation in the perception test mode.

As described above, a perception test can be performed in a smaller number of times of application, and therefore the perception test can be more efficiently performed.

Even in a test site where many subjects exist and hence it is noisy, moreover, a subject can visually recognize the light emission of the light emitter 21, and therefore surely recognize that electrical stimulation is to be applied, without being interrupted by ambient noise.

In the perception test apparatus 1 of the embodiment, moreover, the time interval between the timing of the light emission by the light emitter 21, and that of an application of electrical stimulation by the electrode 10 can be changed. Therefore, the light emission and the application of electrical stimulation can be performed at timings which are appropriate for the subject to perceive electrical stimulation for learning.

Moreover, the perception test apparatus 1 of the embodiment has the stimulation learning mode in which notification is performed. In advance of execution of the perception test, therefore, it is possible to learn the kind of electrical stimulation, and an adequate perception test can be performed. In the perception test mode, electrical stimulation is applied without notification, and therefore the user can be prevented from operating the operating section correspondingly to electrical stimulation which is not perceived, infectiously with notification. Therefore, an adequate perception test can be performed. Moreover, the stimulation learning mode and the perception test mode are switched over, and therefore a perception test can be performed without requiring an additional apparatus dedicated to a perception test.

The perception test apparatus 1 of the embodiment has the push button 22 which is to be operated by the subject in accordance with perception of electrical stimulation. Therefore, the subject can express more surely perception of electrical stimulation.

In the perception test apparatus 1 of the embodiment, in the case where, although electrical stimulation is applied, the push button 22 is not operated, the intensity of electrical stimulation which is to be next applied is raised with reference to the output intensity of the electrical stimulation. Therefore, the output intensity of electrical stimulation for learning can be set in accordance with the degree of the pain sensation of the subject. Consequently, electrical stimulation can be adequately learned.

The perception test method is illustrated as an example and includes: the notifying step (S3) of notifying an application of electrical stimulation to the subject; and the stimulation applying step (S4) of applying electrical stimulation for learning a perception test. Therefore, the subject can be informed of the output of electrical stimulation for learning, regardless of the skill of the inspector.

Although, in the embodiment, the case where the light emitter 21 is disposed as the visual notification section in the projected portion of the switch 20 which is to be gasped by the subject has been exemplarily described, the configuration of the light emitter 21 is not limited to the above-exemplified one. With respect to the shape and size of the light emitter 21, any one of various configurations which can enter the field of vision of the subject can be employed. In the case where the subject learns electrical stimulation while lying on an examination bed, for example, the light emitter 21 may be configured by a light emitter such as a lamp which is disposed in the vicinity of the face of the subject.

The manner of light emission by the light emitter 21 is not limited to that which has been exemplified in the embodiment. In the case where the light emission is conducted three times with respect to one application of electrical stimulation, the colors of the light emissions may be different from one another, or the light intensities of the light emissions may be different from one another. In the case where electrical stimulation is applied a plurality of times at different output intensities, the light emitter 21 may emit light in multiple levels of intensities. For example, the light emitter 21 may emit light in different intensities which correspond respectively to the levels of the output intensity.

Although the case where the light emitter 21 functioning as the visual notification section emits light has been exemplarily described, the manner of the notification by the visual notification section is not limited to light emission. The visual notification section may have any one of various configurations where application of electrical stimulation can be notified to the subject. The notification by the visual notification section may be performed by using a graphic symbol such as a lightning symbol which is displayed on the displaying section 50, characters such as "NOW OUTPUTTING" and "ALREADY OUTPUTTED" which are displayed on the displaying section 50, or a color change such as that a part of the displaying section 50 is displayed in red.

The notification section in the disclosure is not limited to the visual notification section. The notification section in the disclosure may be an audible notification section or a tactile notification section.

The audible notification section may be configured so that the main unit 30 incorporates a speaker (not shown), and the notification may be performed by using a sound produced by the speaker. The notification may be performed by using a sound which is generated for learning of electrical stimulation by a headphone (not shown) that is attached to the subject. The sound functioning as the audible notification section may be a notification sound such as a beep sound, or voice guidance such as "When you perceive pain, please push the push button."

The tactile notification section may be configured so as to vibrate the switch 20, or have a configuration where a heating mechanism (not shown) is incorporated in the switch 20, and the switch 20 is heated.

Although the embodiment which uses one notification section while exemplifying the light emitter 21 has been described above, a configuration where notification is performed b using a plurality of notification sections may be employed.

For example, a configuration may be employed in which the light emitter 21 and the tactile notification section that vibrates the switch 20 are used, and the timing setting section 43 controls so that the light emission by the light emitter 21, and the vibration of the switch 20 are simultaneously performed. When notification is performed by a plurality of notification sections as described above, it is possible to more surely notify the subject of an application of electrical stimulation.

When a plurality of notification sections are used, an application of electrical stimulation for learning can be notified in accordance with the condition of the subject. For example, notification to a subject having hearing impairment may be performed by light emission by the light emitter 21, and vibration of the switch 20. Moreover, notification to a subject having visual impairment may be performed by a sound generated by the headphone, and vibration of the switch 20 which is the operating section. When notification is performed in accordance with the condition of the subject as described above, electrical stimulation can be adequately learned irrespective of the handicap of the subject.

In the embodiment, the notification section is placed separately from the displaying section 50. The manner of placement of the notification section is not limited to the above-described one. A configuration where at least one of the notification section and the operating section is included in the displaying section 50 may be employed. An example in which the notification section is included in the displaying section 50 has a configuration where notification is performed by using a lightning symbol, and the lightning symbol is displayed on the displaying section 50.

The controller 40 may control various settings which are determined in the stimulation learning mode, so as to be used as settings in the perception test mode. Namely, the stimulation controller 41 may set the current value of electrical stimulation which is set in the stimulation learning mode, as that of electrical stimulation in the perception test mode. Moreover, the stimulation controller 41 may reuse set values which, in the stimulation learning mode, are determined as the output intensity of the electrical stimulation which is to be next applied, as set values of the perception test, and use the set values as those of the output intensity in the perception test mode. Moreover, the timing setting section 43 may set the timing of an application of electrical stimulation which is set in the stimulation learning mode, as that of an application of electrical stimulation in the perception test mode. In the configuration where various set values in the stimulation learning mode are reused as set values in the perception test mode as described above, the perception test can be performed adequately and efficiently on the same subject in accordance with the degree of perception of pain of the subject which is acquired in the stimulation learning mode.

The setting in the stimulation learning mode, that in the perception test mode, and the reuse of various set values in the stimulation learning mode, in the perception test mode can be performed automatically or manually.

In the case where, in the stimulation learning mode, the subject perceives a current value of 0.2 mA, for example, the controller 40 may be set to the same value as the current value in the stimulation learning mode, as the initial current value in the perception test mode. When a value which can be perceived by the subject is set as the initial current value in the perception test mode as described above, an adequate perception test can be performed without applying electrical stimulation in which the current value is high (for example, 1.0 mA).

The current value in the perception test mode may be set to a value (for example, 0.25 mA) which is higher than the current value of 0.2 mA that is perceived in the stimulation learning mode. When a current value of 0.25 mA which is higher than a value (the current value of 0.2 mA) that can be perceived by the subject is set as the initial value as described above, and the perception test is performed while gradually lowering the current value from the initial value, it is possible to efficiently detect the maximum value that can be perceived by the subject.

The current value in the perception test mode may be set to a value (for example, 0.18 mA) which is lower than the current value of 0.2 mA that is perceived in the stimulation learning mode. When a current value of 0.18 mA which is lower than a value (the current value of 0.2 mA) that can be perceived by the subject is set as the initial value as described above, and the perception test is performed while gradually raising the current value from the initial value, it is possible to efficiently detect the minimum value that can be perceived by the subject.

When the setting in the perception test mode is performed in accordance with the setting of at least the stimulation controller 41 in the stimulation learning mode as described above, it is possible to perform more efficiently the perception test.

The controller 40 is not limited to have the above-described configuration where it has the stimulation controller 41, the notification controller 42, the timing setting section 43, and the mode switching section 44. The controller 40 may have any one of various configurations where electrical stimulation can be output from the electrode 10, instructions for notification can be output to the notification section, and an operation performed on the operating section can be acquired.

Moreover. various set values of the controller 40 are stored in, for example, a storage apparatus such as an HDD (Hard Disk Drive) in a personal computer, or a storage medium which can be read by a computer, such as an optical disk.

The controller 40 may be connected wiredly or wirelessly to the personal computer, and various data of the controller 40 may be transmitted to the personal computer. The controller 40 may receive various data which are stored in or downloaded to the personal computer.

The settings of the timing of an application of electrical stimulation, and that of notification which are performed by the timing setting section are not limited to the above-described settings. The timing of notification by the notification section may be any one of various timings which are suitable for the subject to know that there is electrical stimulation for learning.

Various timings which are suitable for the subject to know that there is electrical stimulation for learning are determined by setting the interval of timings of notification by the notification section or changing the setting, with reference to the timing of an application of electrical stimulation.

Hereinafter, an example in which the interval of timings of light emission by the light emitter 21 is set with reference to the timing of an application of electrical stimulation will be described.

The light emitter 21 can be configured so as to emit light before the timing of an application of electrical stimulation. For example, the timing setting section 43 is set to minus 0.1 second and minus 1 second.

The light emitter 21 can be configured so as to emit light at the same timing as that of an application of electrical stimulation. For example, the timing setting section 43 may be set to zero second (the light emission and the output of the electrical application are at the same time).

The light emitter 21 can be configured so as to emit light after the timing of an application of electrical stimulation. For example, the timing setting section 43 is set to plus 0.1 second and plus 1.0 second.

The light emitter 21 may be configured so as to emit light between a timing which is a predetermined time before the timing of an application of electrical stimulation, and that when the application of electrical stimulation is ended. For example, the light emitter 21 may emit light from a timing which is 2 seconds before an application of electrical stimulation. The light emission may be performed one time or a plurality of times until stimulation.

The light emitter 21 can be configured so as to stepwise emit light between a timing which is a predetermined time before the timing of an application of electrical stimulation, and that when the application of electrical stimulation is ended. For example, the timing setting section 43 may perform setting in the following manner. The light emission starting is set to minus 2.0 seconds, the number of times of light emission is set to three, the manner of light emission is set to three blinks, and the intervals between the light emissions are set respectively to 0.5 seconds, 0.4 seconds, and 0.3 seconds so as to be gradually shortened. At a timing when electrical stimulation is applied after the third light emission is turned off, it is possible to cause the light emitter 21 to emit light. Since the light emission blinks three times, and the intervals between the light emissions are gradually shortened, countdown for prior notice of electrical stimulation, and warning against approaching of the timing of an application of electrical stimulation is done, thereby enabling the subject to be surely informed of prior notice of an application of electrical stimulation.

The light emitter 21 may be configured so as to emit light between a timing which is a predetermined time before the timing of an application of electrical stimulation, and that which is a predetermined time after the timing of the application of electrical stimulation. For example, the timing setting section 43 may perform setting in the following manner. The light emission is done one time, and the light emission time period is extended from minus 2.0 seconds with respect to an application of electrical stimulation to plus 2.0 seconds. The light emission is started at 2.0 seconds before an application of electrical stimulation, whereby the application of electrical stimulation is previously noticed to the subject, and the light emission is continued until 2.0 seconds after the application of electrical stimulation, whereby the time period when electrical stimulation is applied is clearly indicated, and the application of electrical stimulation is surely notified to the subject.

The light emitter 21 may be configured so as to emit light before an application of electrical stimulation and with placing a long time interval with respect to the timing when the application of electrical stimulation is started. For example, the setting of the timing setting section 43 is minus 3.0 seconds, and light is emitted for a short time period (for example, 0.2 seconds). When the time period between the timing of light emission and that of an application of electrical stimulation is set to be long, the subject is concentrated in an application of electrical stimulation with a sense of perception of applied electrical stimulation, and therefore can learn more surely electrical stimulation.

In the case where electrical stimulation is applied a plurality of times, the timing of light emission of the light emitter 21 may be changed at each light emission. For example, first light emission is performed immediately before (for example, 0.1 second before) an application of electrical stimulation, and, with respect to second light emission, a long time interval (for example, 3.0 seconds before) is placed with respect to an application of electrical stimulation. In the case where the subject expresses that the second electrical stimulation is perceived at the same timing as the first electrical stimulation, it is possible to confirm that the subject is infected with the light emission (notification), and necessary countermeasures can be taken. When the timing when electrical stimulation is applied after light emission is changed at each light emission, the subject can be concentrated in each application of electrical stimulation, and learn more appropriately electrical stimulation. When timings of light emissions are changed, and the subject can accurately perceive electrical stimulation, it is possible to confirm that the subject has learned electrical stimulation without being infected with the notification timing.

The time intervals between the notification by the notification section, and an application of electrical stimulation may be random. For example, the time intervals between the timings of notifications are set through the manual inputting section 60, to be before respective applications of electrical stimulation, and random with respect to the applications.

In the case where the time intervals between the light emission by the light emitter 21, and an application of electrical stimulation are made random, the inspector preferably in advance informs the subject that the time intervals between the light emission and an application of electrical stimulation are random. The subject who recognizes that the time intervals between the light emission and an application of electrical stimulation are random waits with concentration for an application of electrical stimulation after light emission by the light emitter 21, with a sense that electrical stimulation will be perceived. The subject can more adequately learn a reaction to an application of electrical stimulation.

The timing which is adequate for the subject to know that there is electrical stimulation for learning may be determined without reference to the timing of an application of electrical stimulation. In this case, the timing of an application of electrical stimulation is determined by setting of the time interval of timings when the notification section performs notification.

Although, in the embodiment, the setting change in which the output intensity of electrical stimulation is raised has been exemplarily described, the change of the output intensity of electrical stimulation is not limited to the change of raising the output intensity, and a change in which the output intensity is lowered in accordance with the condition of the subject may be performed. In the setting change in which the output intensity of electrical stimulation is lowered, for example, output intensities of electrical stimulation are previously set with reference to the pain sensation of a healthy person, and, in advance of an application of electrical stimulation to a subject who is sensitive to pain, the output intensity of electrical stimulation is lowered to the minimum set value.

Although, in the embodiment, the push button 22 and the light emitter 21 are formed in the same portion of the switch 20, the operating section and the notification section in the disclosure are not limited to have the configuration where they are formed in the same portion. The operating section and the notification section may be configured by individual members. For example, the switch 20 may be formed as a push button (the operating section), and the notification section may be configured by a speaker incorporated in the main unit 30.

A configuration where the operating section and the notification section are included in the displaying section 50 may be employed. In an example in which the operating section and the notification section are included in the displaying section 50, a configuration is employed where the displaying section 50 which is a touch panel is formed as a light emitting section functioning as the notification section, and an operation icon functioning as the operating section is displayed on the touch panel which is the displaying section 50. The subject knows that an electrical application is performed, through light emission of the displaying section 50, and, when the electrical application is perceived, touches the operation icon on the displaying section 50 to express perception of the application of electrical stimulation, so that the electrical stimulation can be learned.

Although, in the embodiment, the operating section is placed separately from the displaying section 50, the placement of the operating section is not limited to the above-described placement. A configuration where the operating section is included in the displaying section 50 may be employed. An example in which the operating section is included in the displaying section 50 has a configuration where the operation icon functioning as the operating section is displayed on the touch panel which is the displaying section 50.

The operating section may be one of various devices through which the subject can express perception of electrical stimulation. For example, a configuration may be employed where a sensor (not shown) which can detect motions of the eyelid and eyeball of the subject is used as the operating section, the subject is instructed so that, when the subject perceives electrical stimulation, the subject closes the eyes, and the sensor detects whether the subject perceives electrical stimulation or not.

The configuration of the perception test apparatus is not limited to that of the embodiment. The perception test apparatus is requested to have a configuration which includes the stimulation controller 41 and the notification section, and the operating section may be omitted. For example, the perception test apparatus which does not have the operating section is configured so that notification from the notification section is displayed on the displaying section 50, and the subject is caused to view and confirm the notification.

The disclosure is not limited to the above-described embodiment and modifications, and may be adequately subjected to modifications, improvements, and the like. In addition, the materials, shapes, forms, number, places, and the like of components of the above-described embodiment are arbitrary and not limited insofar as the disclosure is achieved.

The main unit 30 may be a computer and may include at least a processor such as a CPU (Central Processing Unit), a memory such as a DRAM (Dynamic Random Access Memory) and an auxiliary storage such as a HDD (Hard Disk Drive). Programs for performing the aforementioned processing is stored in the auxiliary storage. The programs in the auxiliary storage is read out by the processor and loaded in the memory when the programs are executed by the processor. By executing the programs by the processor, the aforementioned processing is realized.

For example, sections and controllers (for example, the manual inputting unit 60, the stimulation controller 41, the notification controller 42, the timing setting unit 43 and the mode switching section 44) in the main unit 30 are realized by the processor and the memory.

According to a first aspect of the presently disclosed subject matter, there is provided an apparatus that includes: a stimulation controller configured to control a stimulation application member that applies electrical stimulation to a subject; and a notification section configured to notify the subject of an application of the electrical stimulation.

According to the configuration, the notification section notifies the subject of an application of electrical stimulation, and therefore the subject can be surely informed of an application of electrical stimulation for learning. Since electrical stimulation for learning is applied, the subject can recognize the kind of pain which is produced by the electrical stimulation that is to be used in a perception test.

As an example there is provided a method that includes: notifying of an application of electrical stimulation to a subject; and applying the electrical stimulation for learning a perception test.

According to the method, the application of electrical stimulation for learning is notified, and then the electrical stimulation for learning is applied. Therefore, it is possible to recognize the kind of pain which is produced by the electrical stimulation that is to be used in a perception test.

According to the presently disclosed subject matter, the subject can be informed of the kind of pain which is produced by electrical stimulation that is to be used in a perception test.

## Claims

1. An apparatus (1) for testing perception, comprising:
a stimulation controller (41) configured to control a stimulation application member that applies electrical stimulation to a subject; and
a notification section (21) configured to notify the subject of an application of the electrical stimulation,
wherein the controller is configured to control a stimulation learning mode in which the application of the electrical stimulation is notified by the notification section, and a perception test mode in which the application of the electrical stimulation is performed without performing the notification by the notification section and wherein the stimulation learning mode and the perception test mode are switchable to each other.

2. The apparatus (1) according to claim 1, wherein the notification section (21) comprises at least one of a visual notification section, an audible notification section, and a tactile notification section.

3. The apparatus (1) according to claim 1 or 2, wherein a time interval between a timing when notification by the notification section is performed and a timing when the electrical stimulation is applied is changeable.

4. The apparatus (1) according to any one of claims 1 to 3, further comprising an operating section which is to be operated by the subject in response to perception of the electrical stimulation.

5. The apparatus (1) according to claim 4, wherein the apparatus is configured so that, when an operation corresponding to an application of the electrical stimulation is not performed by the operating section, an intensity of electrical stimulation which is to be next applied is raised with reference to an output intensity of the electrical stimulation.

6. The apparatus (1) according to claim 1, wherein the perception test mode is set in accordance with at least setting of the stimulation controller in the stimulation learning mode.

## Patentansprüche

1. Einrichtung (1) zum Prüfen von Wahrnehmung, umfassend:
eine Stimulationssteuerung (41), konfiguriert, um ein Stimulationsanwendungselement zu steuern, das elektrische Stimulation an einer Versuchsperson anwendet; und
einen Mitteilungsabschnitt (21), konfiguriert, um der Versuchsperson eine Anwendung der elektrischen Stimulation mitzuteilen,
wobei die Steuerung konfiguriert ist, um einen Stimulations-Lernmodus, in dem die Anwendung der elektrischen Stimulation durch den Mitteilungsabschnitt mitgeteilt wird, und einen Wahrnehmungs-Prüfungsmodus zu steuern, in dem die Anwendung der elektrischen Stimulation durchgeführt wird, ohne die Mitteilung durch den Mitteilungsabschnitt durchzuführen und wobei der Stimulations-Lernmodus und der Wahrnehmungs-Prüfungsmodus untereinander umschaltbar sind.

2. Einrichtung (1) nach Anspruch 1, wobei der Mitteilungsabschnitt (21) mindestens einen von einem visuellen Mitteilungsabschnitt, einem akustischen Mitteilungsabschnitt und einem taktilen Mitteilungsabschnitt umfasst.

3. Einrichtung (1) nach Anspruch 1 oder 2, wobei ein Zeitintervall zwischen einem Zeitpunkt, wenn Mitteilung durch den Mitteilungsabschnitt durchgeführt wird, und einem Zeitpunkt, wenn die elektrische Stimulation angewendet wird, veränderbar ist.

4. Einrichtung (1) nach einem der Ansprüche 1 bis 3, weiter umfassend einen Betätigungsabschnitt, der von der Versuchsperson als Reaktion auf Wahrnehmung der elektrischen Stimulation zu betätigen ist.

5. Einrichtung (1) nach Anspruch 4, wobei die Einrichtung so konfiguriert ist, dass, wenn eine einer Anwendung der elektrischen Stimulation entsprechende Betätigung nicht durch den Betätigungsabschnitt durchgeführt wird, eine Intensität von elektrischer Stimulation, die als nächstes anzuwenden ist, mit Bezug auf eine Ausgabeintensität der elektrischen Stimulation erhöht wird.

6. Einrichtung (1) nach Anspruch 1, wobei der Wahrnehmungs-Prüfungsmodus mindestens in Übereinstimmung mit Einstellung der Stimulationssteuerung in dem Stimulations-Lernmodus eingestellt ist.

## Revendications

1. Appareil (1) permettant de tester la perception, comprenant :
un dispositif de commande de stimulation (41) conçu pour commander un élément d'application de stimulation qui applique une stimulation électrique à un sujet ; et
une section de notification (21) conçue pour notifier le sujet d'une application de la stimulation électrique,
dans lequel le dispositif de commande est conçu pour commander un mode d'apprentissage de stimulation dans lequel l'application de la stimulation électrique est notifiée par la section de notification, et un mode de test de perception dans lequel l'application de la stimulation électrique est réalisée sans réaliser la notification par la section de notification et dans lequel le mode d'apprentissage de stimulation et le mode de test de perception peuvent être commutés entre eux.

2. Appareil (1) selon la revendication 1, dans lequel la section de notification (21) comprend au moins une parmi une section de notification visuelle, une section de notification sonore et une section de notification tactile.

3. Appareil (1) selon la revendication 1 ou 2, dans lequel un intervalle de temps entre une synchronisation lorsque la notification par la section de notification est réalisée et une synchronisation lorsque la stimulation électrique est appliquée est modifiable.

4. Appareil (1) selon l'une quelconque des revendications 1 à 3, comprenant en outre une section d'actionnement qui doit être actionnée par le sujet en réponse à la perception de la stimulation électrique.

5. Appareil (1) selon la revendication 4, dans lequel l'appareil est configuré de sorte que, lorsqu'un actionnement correspondant à une application de la stimulation électrique n'est pas réalisé par la section d'actionnement, une intensité de la stimulation électrique qui doit être appliquée ensuite est augmentée par rapport à une intensité de sortie de la stimulation électrique.

6. Appareil (1) selon la revendication 1, dans lequel le mode de test de perception est réglé conformément au moins au réglage du dispositif de commande de stimulation dans le mode d'apprentissage de stimulation.
